# FASCICULE DE BREVET EUROPEEN

(11) **EP 0 747 065 B1**
(45) Date de publication et mention de la délivrance du brevet: **11.07.2001**
(21) Numéro de dépôt: 96470006.6
(22) Date de dépôt: 25.04.1996
(51) Int. Cl.: A61L 15/00, D04H 13/00, C08G 63/16

(54) **Structure composite biodégradable non-tissé/film**
Biologisch abbaubares Vlies/Filmverbundmaterial
Biodegradable non-woven/film laminate

(30) Priorité: 26.04.1995 FR 9505186
(43) Date de publication de la demande: 11.12.1996
(73) Titulaire: FIBERWEB FRANCE SA, 68600 Biesheim (FR)
(72) Inventeur: Ehret, Philippe, 68320 Fortschwihr (FR); Lahteenkorva, Kimmo, 68240 Kaysersberg (FR)
(74) Mandataire: Poupon, Michel

(56) Documents cités:
- WO-A-94/00293
- DE-A- 4 300 420
- DATABASE WPI Section Ch, Week 9512 Derwent Publications Ltd., London, GB; Class A23, AN 95-084400 XP002012214 & JP-A-07 008 520 (MITSUI TOATSU CHEM) , 13 Janvier 1995

## Description

La présente invention concerne une structure composite comprenant une ou plusieurs couches de nontissé associées à un ou plusieurs films à base de polyester.

L'invention concerne également le procédé de fabrication de ladite structure composite et la méthode pour associer les différentes couches qui la constituent.

Habituellement, la ou les couches de nontissé sont utilisées comme support et sont obtenues par un procédé de filature continue ou "spun-bond" désigné par l'abréviation SB.

On ajoute un film destiné à apporter l'étanchéité et l'effet barrière et/ou on ajoute une autre couche de nontissé SB ou de nontissé obtenu par un procédé fondu-soufflé ou "melt-blown" désigné par l'abréviation MB.

Conventionnellement, lesdits produits composites se composent de polyoléfines. Les applications dans lesquelles ce type de composition est utilisé sont : couches-bébé, serviettes périodiques, vêtements de protection, masques et champs opératoires dans le domaine médical et quelques produits pour l'absorption et la filtration, la protection des plantes en agriculture et le paillage.

Les produits à usage unique et jetables sont fabriqués avec des polymères qui sont souvent des matières stables ne se dégradant pas dans la nature.

La quantité de déchets s'accroît continuellement sur toute la planète et l'environnement est de plus en plus pollué. Les produits usagés tels que les films, nontissés pour l'hygiène (couches-bébé, serviettes périodiques...), pour les applications médicales (casaques, champs opératoires...) et pour l'agriculture (protection contre le gel, paillage) constituent une grande partie de ces déchets solides.

L'utilisation de polymères dégradables, en particulier ceux qui sont biodégradables, constitue une grande partie des déchets solides.

Il s'agit principalement de polymères dérivés d'acide lactique (PLA), (Boehringer Ingelheim : RESOMER), de polyester aliphatique thermoplastique (Showa Highpolymer Co. : BIONOLLE ①), de polycaprolactone (PCL), (Union Carbide : TONE, Interox Chemicals : CAPA), de polyhydroxybutyrate/valerate (PHB/V), (Zeneca Bio Products : BIOPOL), d'acide polyglycolide (PGA) et de nombreux polymères et copolymères.

La demande EP 93303000.9 "Biodegradable disposable diaper" déposée par Showa Denko concerne une couche-bébé totalement biodégradable utilisant un nontissé de polyester biodégradable comme voile de surface perméable et comme couche arrière imperméable. La demande PCT/US92/00229 "Novel polyesters and their use in compostable products such as disposable diapers" déposées par E.I. Dupont de Nemours and company concerne plusieurs types d'applications à usage unique comprenant des fibres, films, mousses, etc... à base de nouveaux polyesters biodégradables.

Ces polymères sont bien connus actuellement dans le domaine médical. Ils ont été utilisés comme matière première pour les sutures ; on peut se référer par exemple à : D.E. Cutright et all. dans son traité 'Histologie comparison of polylactic and polyglycolic acid sutures", Oral Surg. 32, 165-173, 1971, pour différents types d'implants (vis, tiges, plaques) ; "polylactic acid for surgical implants publié en 1966 par R.K. Kulkarni et all. Arch.Surg., 93, 839-843 ; Eds et all décrit plusieurs systèmes de diffusion contrôlée d'un principe actif dans "Biodegradable polymers as drug delivery systems : manufacturing methodology, release control and targeting prospects", J. Bioactive Compatible Polymers 1990, 5, 315-342 ; ainsi que la société finlandaise Bioscience Ltd qui fabrique des vis, clous, tiges et plaques (BIOFIX ①), destinés à consolider les fractures osseuses, à base de polymères dérivés d'acide lactique et d'acide glycolique.

Les polymères dérivés de l'acide lactique semblent les plus prometteurs pour remplacer les polymères stables utilisés jusqu'à présent dans la composition des films et nontissés. Les PLA offrent la possibilité d'obtenir des propriétés mécaniques et physicochimiques qui sont comparables à celles des polymères conventionnels (Brevets US 4,743,257, 1988 et 4,968,317, 1990).

La réalisation de structures composites totalement biodégradables comportant des couches de nontissé et de films à base de PLA est cependant difficile à réaliser.

Un but de l'invention est de résoudre ce problème.

En effet, il est très difficile de produire un film fin à base de PLA.

En outre, étant donné la température relativement basse de recristallinisation (90-110°C) des polymères à base de PLA, la lamination ou l'enduction des nontissés avec des films à base de PLA pose des problèmes c'est le cas en particulier de la structure composite comprenant un film imperméable à l'eau et un non-tissé perméable à l'eau faits à base d'acide lactique comme décrit dans JP A 07 008 520. Les films normalement à base de PLA ont également une tendance à être rigides et sont moins maniables que les films à base de polyoléfines, polyamides ou polyesters.

La demanderesse a donc eu l'idée de combiner des couches de nontissé à base de PLA à des films à base de polyester aléphatique biodégradable, en abrégé BAPE.

Un film à base de BAPE présente des propriétés mécaniques semblables au film à base de LDPE, spécialement l'allongement est meilleur que celui du film à base de PLA (BIONOLLE①) : >300 %, PLA : 50-150 %). Il est également totalement compatible avec des nontissés à base de PLA, ce qui permet de les calandrer sans problème : il n'est pas nécessaire d'utiliser le collage et l'enduction par extrusion est possible. La température de fusion (100-200°C) relativement basse permet également de préserver la structure du nontissé lors du calandrage. L'utilisation combinée de ces deux polymères a un effet sur les propriétés de dégradation du composite : la durée de la phase de dégradation est inférieure à celle observée avec des couches comprenant uniquement du PLA ou du BAPE Le polymère de type BAPE présente également une bonne perméabilité à l'air et à l'oxygène, meilleure que celle des PET et LLDPE, ce qui est primordial pour les applications dont la perméabilité à l'air est l'une des spécifications (vêtements de protection, voile arrière pour couche-bébé et plusieurs types d'emballages).

Et, en plus de leur propriété dégradable, les polymères dérivés de PLA sont fabriqués à partir de matières renouvelables telles que le sucre de betterave ou le petit lait. Ainsi la fabrication de ces polymères n'affecte en rien l'équilibre naturel (effet de serre) et n' utilise pas de pétrole, énergie non renouvelable.

L'invention a donc résolu les problèmes de l'art antérieur grâce à une structure composite comprenant une ou plusieurs couches de nontissé et une ou plusieurs couches de film, fabriquées à partir de matières thermoplastiques, caractérisée en ce que toutes les couches de nontissé qui la composent sont totalement fabriquées à partir d'un polymère ou d'un copolymère ou d'un mélange de polymères dérivés d'acide lactique et que toutes les couches du film sont totalement fabriquées à partir d'un polymère du type BAPE (polyester aliphatique biodégradable).

Selon la variante de réalisation utilisée, le polymère dérivé d'acide lactique est un acide lactique D ou un acide lactique L, ou un copolymère d'acide lactique D et L, ou un mélange d'acide lactique L et d'acide lactique D.

Préférentiellement, le polymère, copolymère ou mélange de polymère à un poids moyen moléculaire compris entre 10.000 et 1.000.000.

En outre, le polymère utilisé pour la couche de film est préférentiellement le BIONOLLE. Ledit polymère utilisé pour la couche de film pourra être produit par une réaction chimique de polymérisation entre des glycols et des acides aliphatiques dicarboxyliques et d'autres, par exemple du succinate de polyethylène (PESU).

Selon des modes de réalisations préférentiels, toutes les couches de nontissés qui composent l'invention sont fabriqués par un procédé choisi dans le groupe (SB, voie sèche, MB) et/ou toutes les couches de film sont réalisées par extrusion.

Toutes les couches de nontissés sont soudées ensemble et soudées aux couches de film par thermosoudure, aiguilletage, jets d'eau, over-blowing ou liant chimique.

Toutes ces couches peuvent être soudées ensemble su ligne ou séparément.

On comprendra mieux l'invention à l'aide de la description ci-après faite en référence aux figures annexées suivantes :
- Figure 1 : schéma d'une installation de filature du type "spund-bond" ou SB,
- Figure 2 : schéma d'une installation de fabrication d'une nappe par un procédé de type fondu-soufflé ou MB.

La présente invention concerne une structure composite biodégradable comprenant une ou plusieurs couches de nontissé à base de polymère dérivé d'acide polylactique (PLA), associées à un ou plusieurs films à base de polyester aliphatique (BAPE) ayant une structure chimique identique à celle du BIONOLLE①:

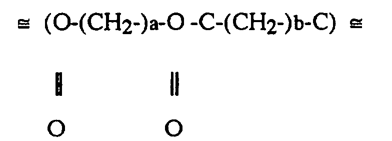

Il est produit par réaction chimique de glycols avec des acides dicarboxyliques aliphatiques et d'autres. Par exemple, il peut s'agir de succinate de polyethylene, PESU (a=2) et de polybutylene succinate PBSU (b=4) Il a une densité se situant entre 1.2 et 1.3 g/cm3. Il est soluble dans l'eau, l'alcool, l'acétone, etc...

Chaque couche possède des propriétés mécaniques : effet barrière, absorption, filtration et isolation thermique pouvant être adaptées à chaque application en sélectionnant la composition adéquate de nontissés à base de PLA et de films à base de BAPE.

Les polymères à base de PLA se composent de L polylactide pur (PLLA), de D polylactide pur (PDLA), de copolymères de polylactide D-L dont le ratio varie de 0 % à 100 %, ainsi que de mélanges des polymères mentionnés.

Le poids moléculaire moyen du polymère peut varier de 10.000 à 1.000.000, de préférence de 40.000 à 600.000. Une dégradation importante s'effectuant pendant la fusion, le poids moléculaire ne devra pas être inférieur.

Le poids moléculaire d'un polymère dérivé de BAPE pourrait se situer entre 10.000 et 600.000 ou de préférence de 20.000 à 300.000.

Les matières premières, (polymères dérivés de PLA et de BAPE), utilisées dans cette invention peuvent contenir un plastifiant dans des proportions de 0.1 à 15 % et/ou de 0.1 à 20 % de monomère lactique et/ou de 0.01 à 5 % de différents types de stabilisants, pigments et autres colorants.

Chaque couche dudit produit composite à plusieurs couches pourra être réalisée en utilisant différentes compositions de matières premières à base de PLA et différents additifs. Chaque couche de film est réalisée à partir de polymère dérivé de BAPE.

Ledit composite se compose d'au moins une couche. Chacune de ces couches est soit un voile de nontissé, soit un film, de sorte que le composite comporte au moins un nontissé. Chaque couche est destiné à un usage spécifique. Dans le cas d'un champ opératoire par exemple, ledit composite comporte une couche de nontissé ayant des propriétés d'absorption et de confort, et une couche de film avec effet barrière (barrière virale). Ledites compositions, particulièrement MB et SB, peuvent également être utilisées dans les serviettes périodiques (brevet US 5,478,069, 1986 utilisant un composite basé sur des polyoléfines) ou des feuillets de stérilisation (brevet US 4,766,029, 1988 utilisant un trilaminé SB+MB+SB).

Dans la présente invention, le voile de nontissé est une matière analogue à un tissu, fabriquée sans tissage ni tricotage, ayant une structure de fibres orientées. Les fibres peuvent être soit continues, soit d'une longueur variant de 5mm à 500mm. De préférence, le titre des fibres se situe de 0.1 à 100 µm.

Le(s) voile(s) peut (peuvent) être soudé(s) ou non soudé(s). La liaison peut s'effectuer par aiguilletage, jets d'eau, liant chimique ou thermosoudage.

### a) Fabrication du nontissé

Il existe différents procédés de type SB pour fabriquer des voiles de nontissés tels que Lurgi, S-Tex et le procédé MB (fondu-soufflé).

La nappe SB apporte de bonnes propriétés mécaniques à la structure composite (résistance, allongement, souplesse). Elle peut également être utilisée pour l'absorption et la filtration. Dans certaines applications où elle constitue la couche de surface, elle apporte la douceur et le confort.

Dans ce procédé le polymère est fondu et extrudé au moyen d'une extrudeuse simple ou à double vis, à une température qui se situe de préférence entre 140 et 280 °C et est acheminé vers une pompe de filature avant de passer à travers un filtre jusqu'à une filière comportant des trous variant de 0.2 à 2.0 mm; de préférence de 0.4 à 1.0 mm. Le polymère est filé à travers la filière jusqu'à l'installation de refroidissement et d'étirage. Le refroidissement peut s'effectuer au moyen d'air refroidi à une température variant de préférence entre 0 et 40°C et la vitesse variant de 0.1 à 5 m/s, l'étirage peut s'effectuer par aspiration d'air ou d'air pulsé à travers le système d'étirage. Le système d'étirage peut comporter une fente ou peut être constitué par une série de tubes ou fentes. La vitesse d'air d'étirage se situe de préférence entre 10 et 400 m/s. Dans le système d'étirage, les fibres obtenues ont un diamètre décroissant et une structure orientée. Le taux d'étirage est généralement de 1.1 à 20, de préférence de 2 à 15. Dans la couche SB le titre des fibres se situe de préférence entre 0.5 et 20 dtex, plus particulièrement de 1 à 10 dtex.

Le système de filature est suivi d'un système de dépose qui couche les fibres au hasard sur le tapis. Le tapis achemine la nappe de fibres vers une calandre chauffée à une température variant de préférence de 40 à 160 °C, plus particulièrement de 60 à 110°C. Avant le calandrage, les autres nappes seront associées pour former le composite, par exemple : une autre nappe SB, une nappe MB ou les deux et/ou une couche de film.

Le poids de base pourra être ajusté selon la vitesse. Il se situe généralement entre 5 à 200 g/m2 en fonction de l'application.

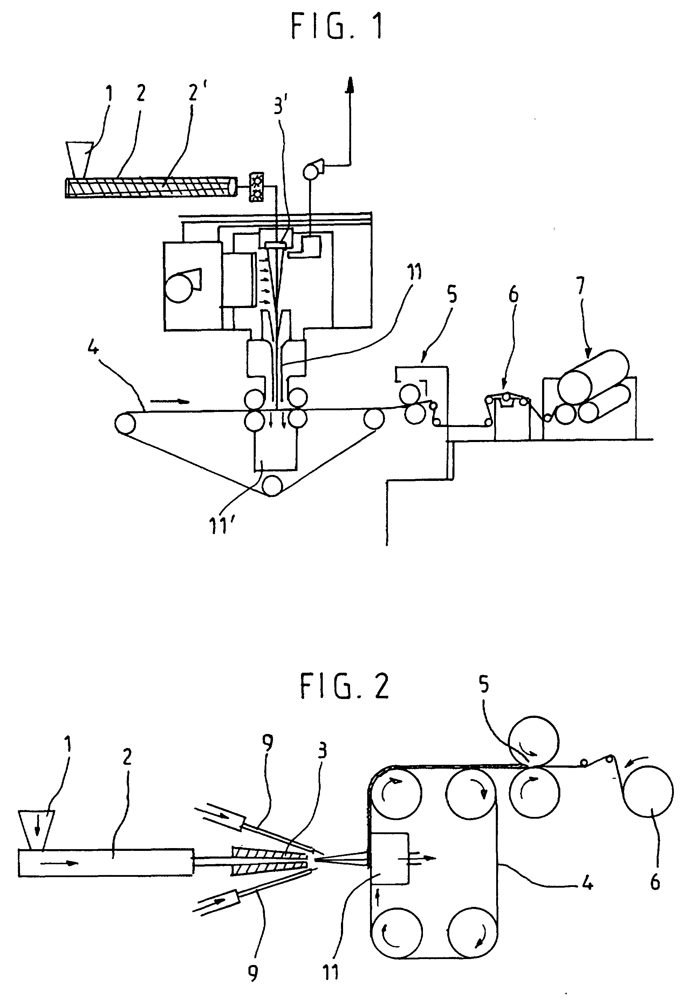

Le schéma de la figure 1 concerne une présentation du procédé SB (i.e. S-Tex) : 1) la trémie pour matière première, 2) l'extrudeuse, 2a') la vis, 3') la filière, 4) le tapis, 5) la calandre, 6) le système de guidage de la nappe et de réglage de tension d'enroulement, 7) l'enroulement, 9) le système de refroidissement des fibres, 11) la fente d'étirage, 11') l'aspiration de l'étirage.

La nappe de ladite structure composite est destinée à apporter des propriétés telles que absorption, filtration et/ou isolation thermique, douceur et confort. Elle permet également d'adapter la perméabilité du composite.

Le procédé MB comprend également une extrudeuse destinée à fondre le polymère. Les températures se situent de préférence entre 150°C et 280°C. Le polymère est véhiculé de l'extrudeuse à la filière. La filière ne comporte qu'une seule rangée de trous. Les trous ont un diamètre de 0.2 à 2 mm.

Un flux d'air des deux côtés de la rangée de trous projette le polymère sous forme de fibres sur la nappe qui défile. Le titre des fibres se situe de 0.05 à 2 dtex. Le poids de base de la nappe MB est ajusté en fonction de la vitesse du tapis.

La nappe MB peut être déposée directement sur la nappe SB. Ce procédé s'appelle "overblowing". Dans ce procédé le système SB est installé avant le système MB, les deux étant installés avant la calandre. Les deux nappes sont thermosoudées par calandrage.

Le schéma de la figure 2 représente le procédé MB : 1) trémie matière première, 2) extrudeuse, 3) filière, 4) tapis de formation, 5) calandre, 6) enrouleuse, 9) soufflage, 11) aspiration.

La couche de film de ladite structure composite est destinée à apporter des propriétés telles que : barrière aux liquides et bonne drapabilité. Le film peut cependant être poreux (brevet US 5,208,098, 1993) et dans ce cas il est perméable et absorbant.

### b) Fabrication du film

Le film BAPE est déposé sur la structure composite par extrusion. La température de l'extrudeuse se situe de 150°C à 220°C et la fente de la filière de 0.2 à 2 mm. La couche de film peut être extrudée séparément et soudée au composite par calandrage, dans ce cas seule une structure nontissée préalablement calandrée pouvant y être associée, soit le film extrudé peut être utilisé pour souder des couches nontissées séparées. L'épaisseur du film ou des films de ladite structure composite se situe de préférence de 0.001 à 1.0mm.

L'utilisation du polymère de type BAPE pour la fabrication d'un film dans ladite structure composite apporte un avantage non négligeable : la température de fusion de ce polymère est inférieure de 40 à 60°C à celle du polymère à base de PLA, ce qui permet de réaliser un laminage, calandrage ou une enduction par extrusion à des températures plus basses, de sorte que le nontissé garde sa résistance et sa douceur.

### c) Fabrication du composite suivant l'invention

Il existe plusieurs manières de fabriquer ladite structure composite selon l'invention, par exemple :
1. Le nontissé composite est lié par thermosoudure, aiguilletage, jets d'eau, over-blowing (MB) ou liant chimique et les films sont associés à cette composition par lamination ou calandrage (figure 3 : 1) nontissé composite, 2) film, calandrage).
2. Le nontissé composite lié par thermosoudure, aiguilletage, jets d'eau, over-blowing (MB) ou liant chimique est associés au(x) film(s) par extrusion/enduction (figure 4 : 1) nontissé soudé, 3) filière d'extrusion, 4) film).
3. Le nontissé est soudé par extrusion/enduction d'un film. Dans le cas de deux ou plusieurs couches de nontissé, le film est extrudé entre les couches afin de les souder et dans le cas d'une seule couche de nontissé cette couche est associée au film par extrusion/enduction (figure 5 : 1-2) nontissé, 3) filière d'extrusion, 4) film).

Un film à base de BAPE associé à un nontissé à base de PLA subit une dégradation plus rapide, lors du compostage ou au cours d'une hydrolyse, qu'un nontissé composite à base de PLA uniquement. Habituellement, une structure composite à base de PLA comprenant un nontissé de 30 g/m2 associé à un film de 10 g/m2 se dégrade en l'espace de 6 à 8 semaines, alors que la dégradation est réalisée en moins d'un mois dans le cas d'un composite à base de PLA et BAPE. Ce composite favorise également la croissance de bactéries et moisissures qui permettent une dégradation plus rapide avec production d'eau et de CO₂.

On décrit ci-après à titre d'exemples non limitatifs, deux structures de nontissé conformes à l'invention.

### Exemple 1 : Structure composite pour l'agriculture

La structure composite destinée à l'usage en agriculture comporte un voile SB à base de PLA et une couche de film à base de BAPE. La structure peut être fabriquée par extrusion/enduction d'une couche de film sur la nappe SB ou en laminant ces deux couches ensemble par thermosoudage. Un colorant noir (noir de carbone 0.5 - 1.5 % adaptée au paillage (brevet US 3,580,196, 1971 utilisant une feuille plastique non dégradable).

Dans l'application pour le paillage, la nappe SB apporte la résistance et le film apporte l'étanchéité. De plus, lorsque la couche SB constitue la face extérieure, celle-ci sèche plus rapidement après la pluie, ce qui permet de conserver les fruits (par exemple : les fraises) et les légumes (par exemple : laitues) en bon état. Par ailleurs le film étanche empêche le dessèchement du sol. La couleur noire constitue un écran au soleil et empêche ainsi la croissance des mauvaises herbes.

En résumé, dans ce cas, la biodégrabilité offre les avantages suivantes : le produit pour paillage peut être laissé en place et il se dégradera avec le temps (fonction modulée de 2 à 36 mois), il peut également être enlevé et enseveli, ou destiné au compostage avec une dégradation de plusieurs semaines.

Dans ladite structure composite, la nappe SB a de préférence un poids se situant de 15 à 75 g/m2 et l'épaisseur du film de 0.01 à 0.10 mm en fonction des spécifications en terme de propriétés mécaniques et de durée de vie. Par exemple, le produit pour paillage contenant 1 % en poids de noir de carbone, ayant un poids de 50 g/m2 pour la nappe SB et une épaisseur de film de 0.025 mm, présente les propriétés suivantes :
- allongement à la rupture : 55-75 %
- résistance sens marche : 110-160 N/m2

La durée de vie pourra être modulée en utilisant un additif stabilisant aux UV.

### Exemple 2 : Structure composite laminée pour application dans l'hygiène

Cette structure composite est composé :
- d'un nontissé à base de PLA ayant un poids de 20 g/m2, un titre de 1.9 dtex, une résistance de 35 N/5cm en sens marche et de 10 N/5cm en sens travers et un allongement de 40 % en sens marche de 50 % en sens travers. Le poids moléculaire moyen est de 100.000 et le pourcentage de monomère est de 0.5 %.
- d'un film BIONOLLES ① d'une épaisseur de 8 µm (≅ 10 g/m2) d'une résistance à la rupture de 300 kg/cm2 et d'un allongement de 300 %.

Ces deux couches sont laminées ensemble pour former une structure composite offrant les propriétés suivantes :
- résistance : 50/5cm sens marche, 25 N/cm sens inverse,
- allongement : 40 % sens marche, 55 % sens travers.
- douceur, toucher textile,
- perméabilité à l'air et à l'eau,
- dégradation totale en compostage en 5 semaines.

Ce composite est utilisable soit en tant que face imperméable d'article d'hygiène (par exemple arrière des couches-bébé) ou comme barrière antifuite dans les couches-bébé et pour incontinent.

De manière générale, la structure composite selon l'invention se caractérise en ce que toutes les couches de nontissé et de films qui la composent offrent chacune des propriétés spécifiques selon le procédé de fabrication, le mode de soudage et le type de polymère sélectionnés.

Enfin, elle peut être adaptée à un produit destiné à l'hygiène (couches-bébé, incontinence, hygiène féminine, etc...) à l'usage en agriculture (paillage, voile de surface) ou au secteur médical (casaques, champs opératoires).

## Revendications

1. Structure composite comprenant une ou plusieurs couches de nontissé et une ou plusieurs couches de film, fabriquées à partir de matières thermoplastiques, caractérisée en ce que toutes les couches de nontissé qui la composent sont totalement fabriquées à partir d'un polymère ou d'un copolymère ou d'un mélange de polymères dérivés d'acide lactique et que toutes les couches du film sont totalement fabriquées à partir d'un polymère du type BAPE (polyester aliphatique biodégradable).

2. Structure composite selon la revendication 1, caractérisée en ce que ledit polymère dérivé d'acide lactique est choisi dans l'ensemble (acide lactique D, acide lactique L).

3. Structure composite selon la revendication 1, caractérisée en ce que ledit polymère dérivé d'acide lactique est un acide lactique D et L.

4. Structure composite selon la revendication 1, caractérisée en ce que ledit polymère est un mélange d'acide lactique L et d'acide lactique D.

5. Structure composite selon la revendication 1, caractérisée en ce que l'élément du groupe (polymère, copolymère, mélange de polymères), a un poids moyen moléculaire compris entre 10.000 et 1.000.000.

6. Structure composite selon l'une des revendications de 1 à 5, caractérisée en ce que le polymère à base de BAPE est le BIONOLLE ①.

7. Structure composite selon l'une des revendications 1 à 6, caractérisée en ce que le polymère utilisé pour la fabrication de chaque film est produit par une réaction chimique de polymérisation entre des glycols et des acides aliphatiques dicarboxyliques et d'autres.

8. Structure composite selon l'une des revendications 1 à 7, caractérisée en ce que l'épaisseur des filaments de chaque couche de nontissé la composant se situe entre 0.1 et 100 µm.

9. Structure composite selon l'une des revendications 1 à 6, caractérisée en ce que l'épaisseur de chaque film la composant se situe entre 0.0001 et 1 mm.

10. Procédé pour la fabrication d'une structure composite selon l'une des revendications 1 à 9, caractérisé en ce que toutes les étapes de fabrication des couches de nontissés sont choisies dans le groupe (SB, voie sèche ou MB).

11. Procédé pour la fabrication d'une structure composite selon l'une des revendications 1 à 10, comprenant une ou plusieurs couches de nontissé et une ou plusieurs couches de film composées de polymères thermoplastiques, caractérisée en ce que la fabrication du film est réalisé par extrusion.

12. Procédé selon l'une des revendications 10 à 11, caractérisé en ce que l'on soude toutes les couches de nontissé ensemble par thermosoudure, aiguilletage, jets d'eau, over-blowing ou liant chimique.

13. Procédé selon l'une des revendications 10 à 12, caractérisé en ce que l'on soude toutes les couches de nontissé et de film par thermosoudure, aiguilletage, jets d'eau, over-blowing, liant chimique ou par extrusion de film.

14. Procédé selon l'une des revendications 10 à 13, caractérisé en ce que l'on soude toutes les couches de nontissé et de film sur ligne ou séparément.

## Patentansprüche

1. Verbundmaterial, welches eine Schicht oder mehrere Schichten aus Vliesstoff und eine Schicht oder mehrere Schichten aus Folie hat, die aus einem thermoplastischen Material hergestellt sind, **dadurch gekennzeichnet,** dass alle Schichten aus Vliesstoff, welche das Verbundmaterial bilden, vollständig aus einem Polymer oder einem Copolymer oder einem Gemisch aus Polymeren hergestellt sind, die aus Milchsäure stammen und dass alle Schichten aus Folie vollständig aus einem Polymer der Art BAPE (biologisch abbaubares, aliphatisches Polyester) stammen.

2. Verbundmaterial nach Anspruch 1, **dadurch gekennzeichnet,** dass das genannte, aus Milchsäure stammende Polymer aus der Gruppe (Milchsäure D, Milchsäure L) ausgewählt ist.

3. Verbundmaterial nach Anspruch 1, **dadurch gekennzeichnet,** dass das genannte, aus Milchsäure stammende Polymer Milchsäure D und L ist.

4. Verbundmaterial nach Anspruch 1, **dadurch gekennzeichnet,** dass das genannte Polymer ein Gemisch aus Milchsäure L und Milchsäure D ist.

5. Verbundmaterial nach Anspruch 1, **dadurch gekennzeichnet,** dass das Element der Gruppe (Polymer, Copolymer, Gemisch aus Polymeren) ein mittleres Molekulargewicht zwischen einschließlich 10.000 und 1.000.000 hat.

6. Verbundmaterial nach einem der Ansprüche 1 bis 5, **dadurch gekennzeichnet,** dass das Polymer auf der Basis von BAPE "BIONOLLE®" ist.

7. Verbundmaterial nach einem der Ansprüche 1 bis 6, **dadurch gekennzeichnet,** dass das zur Herstellung jeder Folie benutzte Polymer durch eine chemische Polymerisationsreaktion zwischen Glykolen und aliphatischen, dicarboxylischen Säuren und anderen erzeugt ist.

8. Verbundmaterial nach einem der Ansprüche 1 bis 7, **dadurch gekennzeichnet,** dass die Dicke der Fasern jeder das Verbundmaterial bildenden Schicht aus Vliesstoff zwischen 0,1 und 100 µm liegt.

9. Verbundmaterial nach einem der Ansprüche 1 bis 6, **dadurch gekennzeichnet,** dass die Dicke jeder das Verbundmaterial bildenden Folie zwischen 0,0001 und 1 mm liegt.

10. Verfahren zur Herstellung eines Verbundmaterials nach einem der Ansprüche 1 bis 9, **dadurch gekennzeichnet,** dass alle Stufen der Herstellung der Schichten aus Vliesstoff aus der Gruppe (SB, trockene Bahn oder MB) ausgewählt sind.

11. Verfahren zur Herstellung eines Verbundmaterials nach einem der Ansprüche 1 bis 10, welches aus einer Schicht oder mehreren Schichten aus Vliesstoff und einer Schicht oder mehreren Schichten aus Folie gebildet ist, **dadurch gekennzeichnet,** dass die Herstellung der Folie durch Extrusion erfolgt.

12. Verfahren nach einem der Ansprüche 10 und 11, **dadurch gekennzeichnet,** dass man alle Schichten aus Vliesstoff durch Thermoschweißen, Vernadelung, Wasserstrahlen, Anblasen oder chemische Bindung miteinander verbindet.

13. Verfahren nach einem der Ansprüche 10 und 12, **dadurch gekennzeichnet,** dass man alle Schichten aus Vliesstoff und aus Folie durch Thermoschweißen, Vernadelung, Wasserstrahlen, Anblasen, chemische Bindung oder durch Extrusion der Folie miteinander verbindet.

14. Verfahren nach den Ansprüchen 10 bis 13, **dadurch gekennzeichnet,** dass man alle Schichten aus Vliesstoff und aus Folie auf der Fertigungslinie oder separat davon verbindet.

## Claims

1. Composite structure, comprising one or more layers of non-woven material and one or more layers of film, manufactured from thermoplastic materials, characterised in that all of the layers of non-woven material, which form its composition, are manufactured entirely from a polymer or from a copolymer or from a mixture of polymers derived from lactic acid, and in that all of the layers of the film are manufactured entirely from a polymer of the BAPE (biodegradable aliphatic polyester) type.

2. Composite structure according to claim 1, characterised in that said polymer, derived from lactic acid, is selected from the group (D lactic acid, L lactic acid ).

3. Composite structure according to claim 1, characterised in that said polymer, derived from lactic acid, is a D and L lactic acid.

4. Composite structure according to claim 1, characterised in that said polymer is a mixture of L lactic acid and D lactic acid.

5. Composite structure according to claim 1, characterised in that the element of the group (polymer, copolymer, mixture of polymers) has a mean molecular weight of between 10,000 and 1,000,000.

6. Composite structure according to one of claims 1 to 5, characterised in that the polymer based on BAPE is BIONOLLE ①.

7. Composite structure according to one of claims 1 to 6, characterised in that the polymer, used for the manufacture of each film, is produced by a chemical polymerisation reaction between glycols and di-carboxylic aliphatic acids and others.

8. Composite structure according to one of claims 1 to 7, characterised in that the thickness of the filaments of each layer of non-woven material forming its composition is between 0.1 and 100 µm.

9. Composite structure according to one of claims 1 to 6, characterised in that the thickness of each film forming its composition is between 0.0001 and 1 mm.

10. Method of manufacturing a composite structure according to one of claims 1 to 9, characterised in that all of the steps for manufacturing the layers of non-woven materials are selected from the group (SB, dry method or MB).

11. Method of manufacturing a composite structure according to one of claims 1 to 10, comprising one or more layers of non-woven material and one or more layers of film composed of thermoplastic polymers, characterised in that the manufacture of the film is effected by extrusion.

12. Method according to one of claims 10 to 11, characterised in that all of the layers of non-woven material are welded together by heat-welding, tagging, water jets, overblowing or chemical bonding.

13. Method according to one of claims 10 to 12, characterised in that all of the layers of non-woven material and of film are welded together by heat-welding, tagging, water jets, overblowing, chemical bonding or by film extrusion.

14. Method according to one of claims 10 to 13, characterised in that all of the layers of non-woven material and of film are welded on line or separately.
